# EUROPEAN PATENT APPLICATION

(11) **EP 2 565 639 A1**
(43) Date of publication of application: **06.03.2013**
(21) Application number: 12183161.4
(22) Date of filing: 05.09.2012
(51) Int. Cl.: G01N 27/407

(54) **Ammonia gas sensor**

(30) Priority: 05.09.2011 JP 2011192572; 17.08.2012 JP 2012180778
(71) Applicant: NGK Spark Plug Co., Ltd., Nagoya-shi, Aichi 467-8525 (JP)
(72) Inventor: Nakano, Yoshihiro, Nagoya-shi, Aichi 467-8525 (JP); Watanabe, Shuntaro, Nagoya-shi, Aichi 467-8525 (JP); Kakimoto, Shiro, Nagoya-shi, Aichi 467-8525 (JP); Sugaya, Satoshi, Nagoya-shi, Aichi 467-8525 (JP); Kojima, Takio, Nagoya-shi, Aichi 467-8525 (JP)
(74) Representative: Grünecker, Kinkeldey, Stockmair & Schwanhäusser

(57) **Abstract**

[Objective] To provide an ammonia gas sensor which is excellent in durability under heating conditions, selectivity to ammonia gas, and detection sensitivity.

[Means for Solution] An ammonia gas sensor 200A having an oxygen ion conductive solid electrolyte member (22A); a detection electrode 2A and a reference electrode 4A which are disposed on the solid electrolyte member; and an intermediate layer 5A interposing between the detection electrode and the solid electrolyte member; wherein the intermediate layer contains the oxygen ion conductive solid electrolyte component in an amount of 50 wt% or more, and a first metal oxide which is an oxide of at least one metal selected from the group consisting of Co, Mn, Cu, Ni, and Ce, and the detection electrode contains Au in an amount of 70 wt% or higher and no first metal oxide.

## Description

### [Technical Field]

The present invention relates to an ammonia gas sensor suitably employed for measuring the ammonia gas concentration (level) of combustion gas or exhaust gas from a combustor, an internal combustion engine, etc.

### [Background Art]

There has been developed a urea SCR (Selective Catalytic Reduction) technique as a method for removing nitrogen oxide (NOₓ) from an exhaust gas from an internal combustion engine of an automobile or the like. In the urea SCR technique, urea is added to an SCR catalyst, to thereby generate ammonia, by which NOₓ is chemically reduced. In the urea SCR technique, an ammonia gas sensor is employed for determining whether or not the level of ammonia for reducing NOₓ is appropriate.

One conventionally proposed ammonia gas sensor includes a solid electrolyte member having oxygen ion conductivity, and a reference electrode and a detection electrode disposed on the surface of the solid electrolyte member, wherein the ammonia concentration is detected by the electromotive force between the electrodes. More specifically, there has been proposed a sensor employing a detection electrode containing gold, and an oxide of a metal such as Al, In, Fe, Cu, Ta, Ga, Sr, Eu, W, Ce, Ti, Zr, or Sn (see Patent Document 1). This sensor can determine the level of combustible gas (e.g., HC gas, CO gas, or ammonia gas) without interference by oxygen concentration in a lean burn engine, where oxygen concentration varies considerably. That is, the selectivity to combustible gas is ensured.
Also, there has been proposed a sensor employing a detection electrode including a solid electrolyte member, a metal (Au) layer formed on the surface of the electrolyte member, and a metal oxide (V₂O₅) layer formed on the metal layer (see Patent Document 2). This sensor can determine the ammonia gas concentration without interference by other gas concentrations and while ensuring the selectivity to ammonia gas by virtue of the metal oxide layer, and the collector performance is ensured by the metal layer.

### [Prior Art Documents]

### [Patent Documents]

[Patent Document 1] Japanese Patent Application Laid-Open *(kokai)* No. 2001-108649
[Patent Document 2] Japanese Patent Application Laid-Open (*kokai*) No. 2008-116321

### [Summary of the Invention]

### [Problems to be Solved by the Invention]

However, the ammonia gas sensor disclosed in Patent Document 1 exhibits a sensitivity to HC gas, CO gas, etc., which is almost equivalent to the sensitivity to ammonia gas. Therefore, the selectivity to ammonia gas is poor, and difficulty is encountered in the selective determination of ammonia gas.
The ammonia gas sensor disclosed in Patent Document 2 has poor durability, which is conceivably caused by poor thermal stability of the metal oxide added to the detection electrode. Particularly, since the temperature of an exhaust gas from an internal combustion engine of an automobile or the like reaches about 700°C, such a sensor is required to have durability against heat.
In addition, when metal oxide is present in the detection electrode or on the surface of the detection electrode, ammonia gas may be burnt before the gas reaches the surface of the solid electrolyte member, thereby lowering the detection sensitivity to ammonia.
In view of the foregoing, an object of the present invention is to provide an ammonia gas sensor which is excellent in durability under heating conditions, selectivity to ammonia gas, and detection sensitivity.

### [Means for Solving the Problems]

In order to solve the aforementioned problems, the present invention provides an ammonia gas sensor having an oxygen ion conductive solid electrolyte member, a detection electrode and a reference electrode provided on a surface of the solid electrolyte member, and an intermediate layer provided between the detection electrode and the solid electrolyte member, wherein the intermediate layer contains an oxygen ion conductive solid electrolyte component in an amount of 50 wt% or more, and a first metal oxide which is an oxide of at least one metal selected from the group consisting of Co, Mn, Cu, Ni, and Ce, and the detection electrode contains Au in an amount of 70 wt% or more but contains no first metal oxide.
According to the ammonia gas sensor, ammonia passes through the detection electrode and reaches the interface between the detection electrode and the underneath intermediate layer, where ammonia reacts with oxygen ions (electrode reaction). During reaction, the first metal oxide contained in the intermediate layer is present at the interface between the detection electrode and the intermediate layer, whereby single selectivity to ammonia gas can be ensured. The first metal oxide is thought to modify the electrode reaction field. In addition, the ammonia gas sensor has excellent durability against heat. Also, since the detection electrode contains no first metal oxide, burning of ammonia gas in the detection electrode is prevented, and the amount of ammonia gas reaching the interface between the detection electrode and the intermediate layer is prevented, whereby the drop in detection sensitivity can be suppressed. Meanwhile, since the detection electrode has an Au content of 70 wt% or more, the detection electrode can serve as a collector while having gas permeability.

In the ammonia gas sensor of the present invention, the reference electrode is preferably disposed directly on the solid electrolyte member.
In one mode for interposing an intermediate layer between the detection electrode and the solid electrolyte member, the intermediate layer is formed on the entire surface of the solid electrolyte member, and then, the detection electrode is formed in the intermediate layer. In this case, the intermediate layer is present under the reference electrode. When an electrode containing Pt is employed as the reference electrode, since Pt has a high firing temperature, the first metal oxide contained in the intermediate layer provided on the entire surface of the solid electrolyte member may vaporize. That is, the amount of first metal oxide present at the interface between the intermediate layer and the detection electrode may decrease. In order to prevent vaporization of the first metal oxide contained in the intermediate layer, the reference electrode is disposed directly on the solid electrolyte member.

Further, in the ammonia gas sensor of the present invention, the intermediate layer preferably contains the first metal oxide in an amount of 1 to 50 wt%. In this case, the selectivity to ammonia gas can be fully attained. When the first metal oxide content is less than 1 wt%, the aforementioned selectivity to ammonia gas may fail to be sufficiently attained, whereas when the first metal oxide content is in excess of 50 wt%, the solid electrolyte component content of the intermediate layer decreases, and the oxygen ion conductive of the intermediate layer may decrease.

In the ammonia gas sensor of the present invention, the intermediate layer is preferably formed such that the first metal oxide is deposited on the solid electrolyte component. In this case, even when the sensor is operated in a high-concentration gas atmosphere, variation in ammonia sensitivity can be reduced. One conceivable reason for this is as follows. Specifically, in the case where the solid electrolyte component and the first metal oxide are present in the intermediate layer as a simple mixture, the first metal oxide contained in the intermediate layer is slightly reduced, and the first metal oxide assumes microparticles, whereby variation in ammonia gas sensitivity occurs. In contrast, in the case where the first metal oxide is deposited on the solid electrolyte component in the intermediate layer, formation of microparticles of the first metal oxide is prevented by virtue of deposition thereof on the solid electrolyte component, whereby variation in ammonia sensitivity can be reduced.

As used herein, the expression "deposition of the first metal oxide on the solid electrolyte component" refers to a state in which a plurality of first metal oxide microparticles are physically bonded to the surface of one solid electrolyte component particle. The state in which the first metal oxide is deposited on the solid electrolyte component may be confirmed through the following method. Specifically, images of areas having specific dimensions (e.g., 3 µm x 3 µm) are taken from a surface or a cross-section of the intermediate layer under a scanning electron microscope (SEM) or a scanning transmission electron microscope (STEM). The case where the first metal oxide not bonded to the solid electrolyte component is observed in no area, or the case where first metal oxide not bonded to the solid electrolyte component is observed in only one area indicates the state in which the first metal oxide is deposited on the solid electrolyte component. In other words, when the state in which the first metal oxide is not bonded to the solid electrolyte component is not observed in two or more areas, the state in which the first metal oxide is deposited on the solid electrolyte component can be confirmed.
Generally, the first metal oxide assumes the particle form and virtually does not assume an excessively elongated form. Thus, in the case where an elongated form first metal oxide is present in any of the aforementioned images, if the first metal oxide particles surrounding the elongated-form first metal oxide assume aggregates (or are sintered) determined by the form and dimensions thereof, elongated-form first metal oxide which is not bonded to the solid electrolyte component and which is surrounded by the first metal oxide is regarded as a "first metal oxide not bonded to the solid electrolyte component." In the case where an isolated (not aggregated) first metal oxide which is not bonded to the solid electrolyte component is present (i.e., discretely present), the isolated first metal oxide is regarded as a "first metal oxide not bonded to the solid electrolyte component." The state in which a plurality of first metal oxide particles are linked together (are sintered) is not limited to the aforementioned aggregation state. Whether or not the linkage of the first metal oxide is present is determined on the basis of the mean particle size and shape of the particles surrounding (not bonded to) the linked first metal oxide mass (when the linkage of the first metal oxide is present, twisting or cavity is observed between first metal oxide units).

In the ammonia gas sensor of the present invention, when the detection electrode is a porous electrode containing a second metal oxide, which is an oxide of at least one metal selected from the group consisting of Zr, Y, Al, and Si, the detection electrode exhibits sufficient gas permeability. In this case, ammonia passes through the detection electrode and readily reaches the interface between the detection electrode and the underneath intermediate layer. As a result, single selectivity to ammonia gas can be ensured.

In the ammonia gas sensor of the present invention, the first metal oxide is preferably CO₃O₄. When the first metal oxide is CO₃O₄, variation in ammonia sensitivity of the ammonia gas sensor, which would otherwise be caused by H₂O contained in a sample gas ammonia gas sensor, can be reduced.

Also, in the ammonia gas sensor of the present invention, the intermediate layer is preferably porous. When the intermediate layer is porous, gas substitution is promoted, to thereby suppress burning of ammonia gas, and sensitivity to detection of ammonia gas and selectivity to ammonia gas are enhanced, which is preferred.

### [Effects of the Invention]

The present invention enables provision of an ammonia gas sensor which is excellent in durability under heating conditions, selectivity to ammonia gas, and detection sensitivity.

### [Brief Description of the Drawings]

[FIG. 1] Sectional view of an ammonia gas sensor according to an embodiment of the present invention taken along the longitudinal direction thereof.
[FIG. 2] Exploded view showing the structure of a sensor element.
[FIG. 3] Sectional view taken along line III-III of FIG. 2.
[FIG. 4] Graph showing a change in ammonia selectivity depending on the type of first metal oxide contained in an intermediate layer.
[FIG. 5] Graph showing a change in ammonia detection sensitivity depending on the type of first metal oxide contained in the intermediate layer.
[FIG. 6] Graph showing a change in ammonia detection sensitivity with gas flow speed in Example 4.
[FIG. 7] Graph showing a change in ammonia detection sensitivity with gas flow speed in Comparative Example 3.
[FIG. 8] Graph showing the results of evaluation on the applicability to high-concentration gas in Example 3.
[FIG. 9] Graph showing the results of evaluation on the applicability to high-concentration gas in Example 11.
[FIG. 10] Graph showing the results of evaluation on the applicability to high-concentration gas in Example 12.
[FIG. 11] Graph showing the results of evaluation on the applicability to high-concentration gas in Example 13.
[FIG. 12] Graph showing the results of evaluation on the applicability to high-concentration gas in Example 14.
[FIG. 13] Graph showing the results of evaluation on the applicability to high-concentration gas in Example 15.
[FIG. 14] Graph showing the results of evaluation on the applicability to high-concentration gas in Example 16.

### [Mode for Carrying out the Invention]

An embodiment of the present invention will now be described.

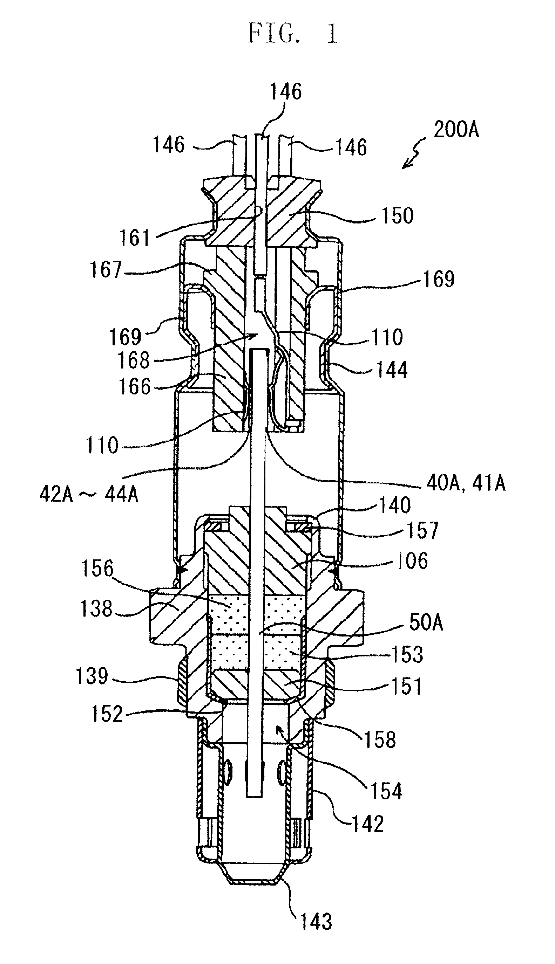

FIG. 1 shows a sectional view of an ammonia gas sensor (ammonia sensor) 200A according to the embodiment of the present invention taken along the longitudinal direction thereof. The ammonia sensor 200A is an assembly which includes a sensor element 50A for detecting ammonia. The ammonia sensor 200A includes the plate-shaped sensor element 50A extending in the axial direction; a tubular metallic shell 138 which has, on its outer surface, a screw portion 139 for fixing the ammonia sensor 200A to an exhaust pipe; a tubular ceramic sleeve 106 which is disposed such that it circumferentially surrounds the sensor element 50A; an insulative contract member 166 which has a contact insertion hole 168 extending therethrough in the axial direction and which is disposed such that the wall surface of the contact insertion hole 168 surrounds the circumference of a rear end portion of the sensor element 50A; and a plurality of connection terminals 110 (only two of them are shown in FIG. 1) disposed between the sensor element 50A and the insulative contract member 166.

The metallic shell 138, which is formed into a generally tubular shape, has a through-hole 154 extending therethrough in the axial direction, and a ledge portion 152 projecting inward in the radial direction of the through-hole 154. The metallic shell 138 holds the sensor element 50A within the through-hole 154 in a state in which a front end portion of the sensor element 50A projects frontward from the through-hole 154, and electrode terminal portions 40A to 44A project rearward from the through-hole 154. The ledge portion 152 is formed as an inward taper surface which inclines in relation to a plane perpendicular to the axial direction.

Within the through-hole 154 of the metallic shell 138, an annular ceramic holder 151, powder layers 153 and 156 (hereinafter also referred to as the "talc rings 153 and 156), and the above-described ceramic sleeve 106 are disposed in this sequence from the front end side toward the rear end side in a state in which they circumferentially surround the sensor element 50A. A crimp packing 157 is disposed between the ceramic sleeve 106 and a rear end portion 140 of the metallic shell 138. A metallic holder 158 is disposed between the ceramic holder 151 and the ledge portion 152 of the metallic shell 138 so as to hold the talc ring 153 and the ceramic holder 151, and maintain air tightness. The rear end portion 140 of the metallic shell 138 is crimped in such a manner that the rear end portion 140 presses the ceramic sleeve 106 frontward via the crimp packing 157.

Meanwhile, as shown in FIG. 1, an outer protector 142 and an inner protector 143 formed of metal (e.g. stainless steel) are attached to the outer periphery of a front end portion (a lower end portion in FIG. 1) of the metallic shell 138 by welding or the like so as to cover the projecting portion of the sensor element 50A. Each of the outer protector 142 and the inner protector 143 has a plurality of holes.

A sleeve 144 is fixed to the outer periphery of a rear end portion of the metallic shell 138. A grommet 150 is disposed in an opening of a rear end portion (an upper end portion in FIG. 1) of the sleeve 144. The grommet 150 has a lead wire insertion hole 161, into which five lead wires 146 (only three of them are shown in FIG. 1) electrically connected to the electrode terminal portions 40A to 44A of the sensor element 50A are inserted.

The insulative contract member 166 is disposed at a rear end portion (an upper end portion in FIG. 1) of the sensor element 50A, which projects from the rear end portion 140 of the metallic shell 138. The insulative contract member 166 is disposed around the electrode terminal portions 40A to 44A formed on the surface of the rear end portion of the sensor element 50A. The insulative contract member 166, which is formed into a tubular shape, has a contact insertion hole 168 extending therethrough in the axial direction, and a flange portion 167 projecting outward from the outer surface in the radial direction. The flange portion 167 is engaged with the sleeve 144 via a holding member 169, whereby the insulative contract member 166 is disposed inside the sleeve 144. The connection terminals 110 held by the insulative contract member 166 are electrically connected to the electrode terminal portions 40A to 44A of the sensor element 50A, whereby the electrode terminal portions 40A to 44A of the sensor element 50A are electrically connected to an external circuit via the lead wires 146.

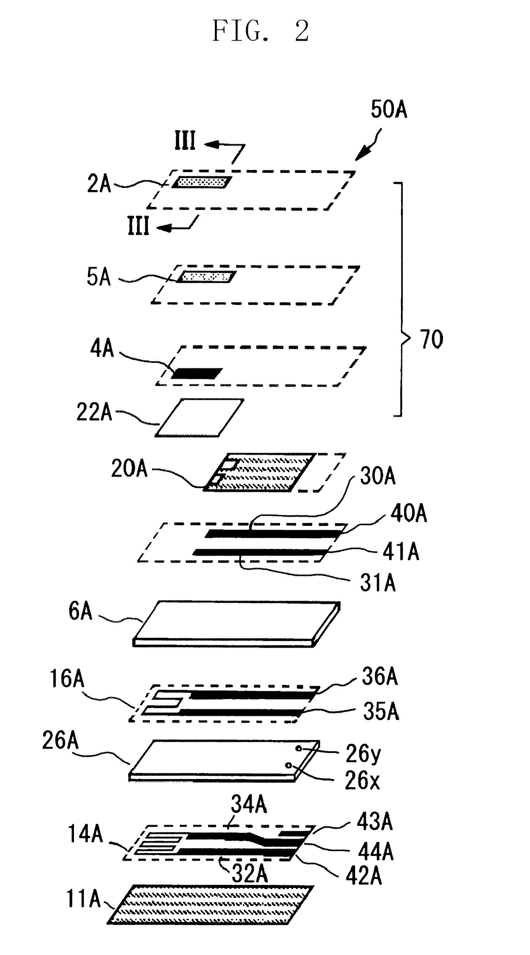

Next, the structure of the sensor element 50A will be described with reference to the exploded view of FIG. 2. The sensor element 50A has a shape of an elongated plate and is configured such that a detection portion for detecting ammonia gas contained in exhaust gas is exposed at the front end portion of the sensor element 50A, and the electrode terminal portions 40A to 44A are exposed at the rear end portion of the sensor element 50A.
As shown in FIG. 2, on the upper surface of the insulation layer 6A, a lead 31A extends in the longitudinal direction, and an end of the lead 31A forms the electrode terminal portion 41A. Further, on the insulation layer 6A, a lead 30A extends in parallel with the lead 31A, and an end of the lead 30A (at the right end of the insulation layer 6A) forms the electrode terminal portion 40A. The leads 30A and 31A extend in the longitudinal direction from a central portion of the insulation layer 6A to the right end thereof. An insulation layer 20A is formed to cover the leads 30A and 31A. However, a front end portion (a portion where the leads 30A and 31A are not formed) of the insulation layer 6A, front end portions of the leads 30A and 31A, and the electrode terminal portions 40A and 41A are not covered by the insulation layer 20A, and are exposed.

Meanwhile, a solid electrolyte member 22A is disposed on a portion of the insulation layer 6A which is not covered by the insulation layer 20A. A rectangular reference electrode 4A is formed on the solid electrolyte member 22A. Furthermore, a rectangular detection electrode 2A is formed on the solid electrolyte member 22A with an intermediate layer 5A interposed therebetween such that the detection electrode 2A becomes parallel to the reference electrode 4A. The reference electrode 4A is connected to the lead 31A, and the detection electrode 2A is connected to the lead 30A. The intermediate layer 5A has a rectangular shape slightly greater than the detection electrode 2A.
As described above, the reference electrode 4A and the detection electrode 2A are provided on the same side of the solid electrolyte member 22A, and are exposed to a gas to be measured. The solid electrolyte member 22A, the reference electrode 4A, the intermediate layer 5A, and the detection electrode 2A constitute a cell 70.

Meanwhile, temperature detection means (a temperature sensor) 14A, which is a temperature measurement resistor, and leads 32A and 34A are formed on the lower surface (the lower surface in FIG. 2) of the insulation layer 26A. An end of the lead 34A forms the electrode terminal portion 44A. Also, the lead 32A extends in parallel with the lead 34A, and an end of the lead 32A form the electrode terminal portion 42A. A heat generation resistor 16A and leads 35A and 36A extending from the heat generation resistor 16A are formed on the upper surface of the insulation layer 26A. The temperature detection means 14A and the leads 32A and 34A are covered by an insulation layer 11A. The heat generation resistor 16A and the leads 35A and 36A are covered by the insulation layer 6A. Through-holes 26x and 26y are formed at the right end of the insulation layer 26A. The leads 35A and 36A are connected, via the through-holes 26x and 26y, to the electrode terminal portion 42A and 43A disposed on the lower surface of the insulation layer 26A.
Notably, a gas permeable protection layer may be provided on either one or both of the detection electrode 2A and the reference electrode 4A.

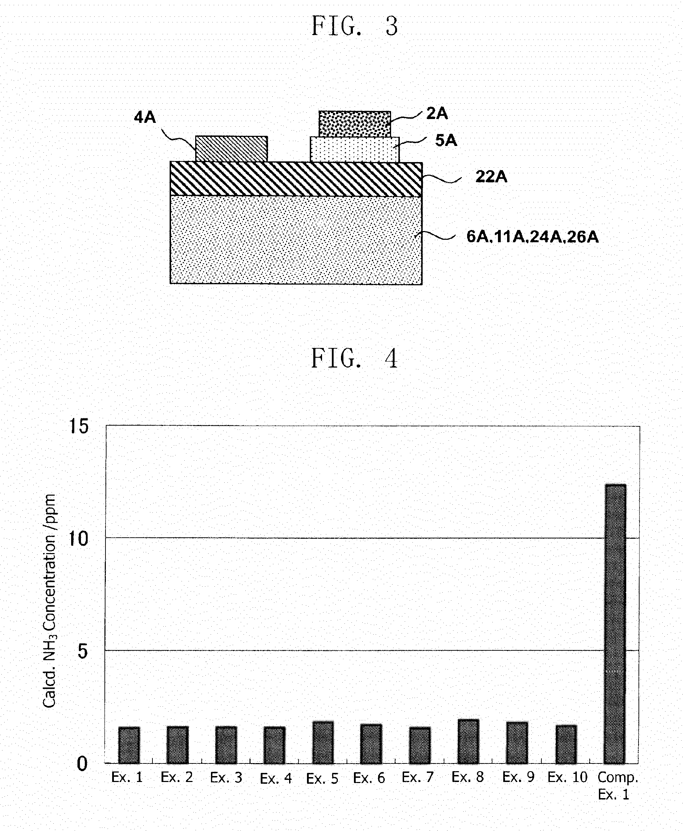

FIG. 3 is a sectional view taken along line III-III of FIG. 2. Notably, in FIG. 3, structural elements, other than the cell 70, are illustrated in a simplified manner.
The detection electrode 2A contains Au in an amount of 70 wt% or greater, and does not contain a first metal oxide to be described later. Therefore, a combustible gas hardly burns on the surface of the electrode. The intermediate layer 5A contains an oxygen-ion-conductive solid electrolyte component in an amount of 50 wt% or greater, and the first metal oxide, which is an oxide of at least one metal selected from the group consisting of Co, Mn, Cu, Ni, and Ce.
Since ammonia passes through the detection electrode 2A and reacts with oxygen ions at the interface between the detection electrode 2A and the underneath intermediate layer 5A (electrode reaction), the detection electrode 2A and the intermediate layer 5A serve as an ammonia gas detection portion. When the first metal oxide is present at the interface between the detection electrode 2A and the intermediate layer 5A, the sensitivity for gasses (HC gas, etc.) other than ammonia gas decreases, and only the sensitivity for ammonia gas increases. Although the mechanism is not clear, conceivably, such a phenomenon occurs because the first metal oxide present at the interface modifies the electrode reaction field. Also, it is considered that, since the first metal oxide has acidity, it strongly interacts with NH₃, which is a basic molecule, and promotes the electrode reaction for NH₃ efficiently, as compared with other gases, whereby ammonia selectivity is enhanced.
Moreover, since the detection electrode 2A does not contain the first metal oxide, burning of ammonia gas within the detection electrode 2A is restrained, and the amount of ammonia gas reaching the interface between the detection electrode 2A and the intermediate layer 5A does not decrease. Therefore, the ammonia sensor has an improved detection sensitivity. In particular, the ammonia sensor can detect ammonia of a low concentration (0 to 10 ppm) with high accuracy.

In place of using the intermediate layer 5A, the first metal oxide may be added to the solid electrolyte member 22 in order to realize the structure in which the detection electrode 2A does not contain the first metal oxide and a member adjacent thereto contains the first metal oxide. However, since the solid electrolyte member 22A is formed of, for example, partially stabilized zirconia, it must be fired at a high temperature (about 1,500°C). Therefore, there is a possibility that the first metal oxide evaporates from the solid electrolyte member 22A during the firing. Accordingly, the intermediate layer 5A is desirably interposed between the solid electrolyte member 22 and the detection electrode 2A as in the present embodiment.

The first metal oxide contained in the intermediate layer 5A is an oxide of at least one metal selected from the group consisting of Co, Mn, Cu, Ni, and Ce. The metal oxide is particularly preferably CO₃O₄, since variation in ammonia sensitivity of the ammonia gas sensor, which would otherwise be caused by H₂O contained in a sample gas, can be reduced. Notably, the first metal oxide assumes a metal oxide or a complex metal oxide. The solid electrolyte component contained in the intermediate layer 5A may have a composition identical to or different from the composition of the solid electrolyte member 22, which is a member of the gas sensor of the present invention.

The intermediate layer 5A preferably contains the first metal oxide in an amount of 1 to 50 wt%. When the first metal oxide content is less than 1 wt%, the aforementioned selectivity to ammonia gas may fail to be fully attained, whereas when the first metal oxide content is in excess of 50 wt%, the intermediate layer 5A has a lower solid electrolyte component content, whereby the intermediate layer 5A may exhibit reduced oxygen ion conductivity.
When the intermediate layer 5A is porous, detection sensitivity and selectivity to ammonia gas are enhanced, which is preferred.
The state in which the first metal oxide is contained in the intermediate layer 5A can be confirmed through EPMA (electron probe microanalysis) of a cross-section of the ammonia gas sensor (generally, average of three analysis sites).

The detection electrode 2A has an Au content of 70 wt% or more, whereby the detection electrode can exhibit collector performance. When the Au content is less than 70 wt%, collector performance cannot be attained, and ammonia gas cannot be detected.
When the detection electrode 2A is a porous electrode containing the second metal oxide, which is an oxide of at least one metal selected from the group consisting of Zr, Y, Al, and Si, the detection electrode can exhibit sufficient gas permeability. In this case, ammonia passes through the detection electrode and readily reaches the interface between the detection electrode and the underneath intermediate layer. As a result, single selectivity to ammonia gas can be ensured. The detection electrode 2A preferably contains the second metal oxide in an amount of 5 to 30 wt%.

The reference electrode 4A is an electrode, and combustible gas burns on the surface of the electrode. The reference electrode is formed of, for example, Pt, or a material predominantly containing Pt.
Preferably, the reference electrode 4A is disposed directly under the solid electrolyte member 22A. In other words, the intermediate layer 5A is preferably absent underneath the reference electrode 4A. In one technique for interposing the intermediate layer 5A between the detection electrode 2A and the solid electrolyte member 22A, the intermediate layer 5A is formed on the entire surface of the solid electrolyte member 22A, and the detection electrode 2A is formed on the intermediate layer 5A. In this case, the intermediate layer 5A is also present underneath the reference electrode 4A. However, when an electrode containing Pt is employed as the reference electrode 4A, since Pt has high firing temperature (about 1,400°C or higher), the first metal oxide contained in the intermediate layer 5A may vaporize in the vicinity of the reference electrode 4A.
When Ce, which is difficult to vaporize, is employed as the first metal oxide, the intermediate layer 5A may be present underneath the reference electrode 4A.

The leads 30A, 31A, 32A, 34A, 35A, and 36A, electrode terminal portions 40A to 44A, temperature detection means 14A, and heat generation resistor 16A are formed of, for example, a material predominantly containing Pt, Pd, or an alloy thereof.
The insulation layers 6A, 11A, 20A, and 26A are formed of, for example, an insulative ceramic material such as alumina.
The solid electrolyte member 22A is formed of, for example, partially stabilized zirconia (YSZ). The solid electrolyte member 22A is maintained at an activation temperature by means of the heat generation resistor 16A.

Next, one exemplary method for producing the sensor element portion 50A will be briefly described. Firstly, a green alumina insulation layer 26A having a relatively large thickness (e.g., 300 µm) and serving as a main body of the sensor element is provided. On the upper surface of the insulation layer 26A, an electrode paste containing Pt, alumina (inorganic oxide serving as a co-base), a binder, and an organic solvent (hereinafter referred to as a "Pt-based paste") is applied through screen printing, to thereby form the heat generation resistor 16A (and leads 35A, 36A extending therefrom). On the lower surface, temperature detection means 14A (and leads 32A, 34A extending therefrom), and electrode terminal portions 42A, 43A, 44A were formed. Also, on the lower surface of the temperature detection means 14A, a paste containing an insulative material (e.g., alumina), a binder, and an organic solvent is applied through screen printing, to thereby form the insulation layer 11A. Notably, a through-hole conductor is appropriately charged into the through-holes 26x, 26y of the insulation layer 26A.

Then, a green alumina insulation layer 6A having a relatively large thickness (e.g., 300 µm) and serving as the main body of the sensor element portion is stacked on the heat generation resistor 16A. On the insulation layer 6A, leads 30A, 31A, and electrode terminal portions 40A, 41A are formed. Further, the insulation layer 20A is screen-printed so as to cover the leads 30A, 31A. Notably, the insulation layer 6A may be formed through screen printing of an insulative paste.

The stacked body is de-bindered at a predetermined temperature (e.g., 250°C) and fired at a predetermined temperature (e.g., 1,400°C).

Then, a paste containing oxide powder, a binder, and an organic solvent, which paste serves as a component of the solid electrolyte member, is applied, through screen printing, onto the insulation layer 6A after firing, to thereby form the solid electrolyte member 22A, followed by firing at a predetermined temperature (e.g., 1,500°C).

Next, onto the solid electrolyte member 22A, a Pt-based paste is applied through screen printing, to thereby form the reference electrode 4A, followed by firing (at, e.g., 1,400°C or higher). Subsequently, a paste containing the aforementioned first metal oxide and solid electrolyte component is applied, through screen printing, onto the reference electrode 4A, to thereby form the intermediate layer 5A. In this case, the paste is prepared from a powder of the first metal oxide deposited on the solid electrolyte component. As a result, in the intermediate layer 5A after firing, the first metal oxide is deposited on the solid electrolyte component. Thus, since the intermediate layer 5A contains the first metal oxide deposited on the solid electrolyte component, even when the ammonia gas sensor is operated in a high-concentration gas atmosphere, variation in ammonia sensitivity can be reduced.
On the intermediate layer 5A, an Au-based paste is screen-printed, to thereby form the detection electrode 2A. The resultant assembly is fired at a predetermined temperature (e.g., 1,000°C), which is relatively lower than the above-employed firing temperature.

The aforementioned embodiment should not construed as limiting the invention thereto, and the invention encompasses various modifications and equivalents, so long as the scope of the invention is not impaired. One modification is a dual-chamber sensor structure, in which a detection electrode is formed on one surface of the solid electrolyte member, and a reference electrode is disposed on the other surface thereof, wherein the reference electrode is operated in air, and the detection electrode is brought into contact with a gas to be analyzed.
Alternatively, in another sensor structure, the solid electrolyte member may be formed into a cylinder, and a detection electrode is disposed on the outer surface of the cylinder, and the reference electrode is disposed on the inner surface thereof. The inner surface is exposed to air, and the detection electrode on the outer surface is exposed to a gas to be analyzed.

### [Examples]

The present invention will next be described in detail by way of examples, which should not be construed as limiting the invention thereto.

An ammonia gas sensor shown in FIGs. 1 and 2 according to the aforementioned embodiment was fabricated. Firstly, a Pt-based paste was applied through screen printing onto the upper surface of an alumina substrate (insulation layer) 26A, to thereby form a heat generation resistor 16A (and leads 35A and 36A extending therefrom), and the Pt-based paste was applied through screen printing onto the lower surface thereof, to thereby form a temperature detection means 14A (and leads 32A and 34A extending therefrom) and electrode terminal portions 42A, 43A, and 44A. Onto the upper surface of the heat generation resistor 16A and the upper surface of the temperature detection means 14A, a paste containing an insulative material, a binder, and an organic solvent was applied through screen printing, to thereby form an insulation layer 11A.
Then, a green alumina insulation layer 6A having a relatively large thickness (e.g., 300 µm) and serving as the main body of the sensor element portion was stacked on the heat generation resistor 16A. On the insulation layer 6A, leads 30A and 31A, and electrode terminal portions 40A and 41A were formed by screen-printing a Pt-based paste. Further, the insulation layer 20A was screen-printed so as to cover the leads 30A, 31A. The stacked body was de-bindered at a predetermined temperature (e.g., 250°C) and fired at a predetermined temperature (e.g., 1,500°C) for 60 minutes.
Subsequently, a YSZ (Y-stabilized zirconia) paste serving as a material of a solid electrolyte member 22A was applied through screen printing onto the insulation layer 6A, and the stacked body was fired at 1,500°C for 60 minutes. The YSZ paste was prepared by adding YSZ, an organic solvent, and a dispersant to a mortar, mixing the mixture for dispersion by means of a Raikai mixer for 4 hours, adding predetermined amounts of a binder and a viscosity-adjusting agent to the resultant mixture, and performing wet mixing for 4 hours.

Then, a Pt-based paste was applied through screen printing onto the solid electrolyte member 22A, to thereby form a reference electrode 4A, followed by firing at 1,450°C. Thereafter, any of the pastes having a composition shown in Table 1 was screen-printed, to thereby form an intermediate layer 5A, and an Au-based paste was applied through screen printing onto the intermediate layer 5A, to thereby form a detection electrode 2A. The stacked body was fired at 1,000°C. The thus-produced sensor element portion was attached to a metallic shell or the like, to thereby fabricate an ammonia gas sensor. The aforementioned Au-based paste was prepared by blending a commercial Au paste with any of the powders listed in Table 1 and homogenizing the mixture for 10 minutes or longer. The paste for forming the intermediate layer 5A was prepared by mixing a solid electrolyte component powder and a first metal oxide powder. Specifically, a predetermined amount of a first metal oxide powder was added to the aforementioned YSZ paste, and the mixture was wet-mixed by means of a Raikai mixer for 4 hours.
The type and amount of the first metal oxide powder in the aforementioned intermediate layer 5A were modified as shown in Table 1, whereby ammonia gas sensors of Examples 1 to 10 and Comparative Examples 1 to 5 were fabricated. In Table 1, YSZ denotes a partially stabilized zirconia.

**[Table 1]**

| | YSZ content of solid electrolyte member | Intermediate layer | | Detection electrode | | | Evaluation | | |
|---|---|---|---|---|---|---|---|---|---|
| | | First metal oxide (type/content) | Solid electrolyte component (type/content) | Au | YSZ | Co₃O₄ | Selectivity | Sensitivity | Flow speed |
| Ex. 1 | 100 wt.% | Co₃O₄ 1 wt.% | YSZ 99 wt. % | 85 wt.% | 15 wt.% | - | O | OO | O |
| Ex. 2 | 100 wt.% | Co₃O₄ 2 wt.% | YSZ 98 wt.% | 85 wt.% | 15 wt.% | - | O | OO | O |
| Ex. 3 | 100 wt.% | Co₃O₄ 5 wt.% | YSZ 95 wt.% | 85 wt.% | 15 wt.% | - | O | OO | O |
| Ex. 4 | 100 wt.% | Co₃O₄ | YSZ 90 wt.% | 85 wt.% | 15 wt.% | - | O | OO | O |
| Ex. 5 | 100 wt. % | Co₃O₄ | YSZ 80 wt. % | 85 wt.% | 15 wt.% | - | O | OO | O |
| Ex. 6 | 100 wt.% | Co₃O₄ 50 wt.% | YSZ 50 wt. % | 85 wt.% | 15 wt.% | - | O | OO | O |
| Ex. 7 | 100 wt.% | MnO 10 wt. % | YSZ 90 wt. % | 85 wt.% | 15 wt% | - | O | OO | O |
| Ex. 8 | 100 wt.% | CuO 10 wt.% | YSZ 90 wt. % | 85 wt. % | 15 wt.% | - | O | OO | O |
| Ex. 9 | 100 w.t% | NiO 10 wt. % | YSZ 90 wt.% | 8 5wt.% | 15wt.% | - | O | OO | O |
| Ex. 10 | 100 wt.% | CeO₂ 10 wt.% | YSZ 90 wt.% | 85 wt.% | 15 wt.% | - | O | OO | O |
| Comp. Ex. 1 | 100 wt.% | - | - | 85 wt.% | 15 wt.% | - | x | - | - |
| Comp. Ex. 2 | 100 wt.% | 100 wt% | - | 85 Wt.% | 15 wt.% | - | - | - | - |
| Comp. Ex. 3 | 100 wt.% | - | - | 75 wt.% | 15 wt.% | 10 wt.% | - | O | x |
| Comp. Ex. 4 | 100 Wt% | Co₃O₄ 10 wt.% | YSZ 90wt.% | 60 wt.% | 40 wt.% | - | - | - | - |
| Comp. Ex. 5 | 100 wt.% | Co₃O₄ 60 w.% | YSZ 40 wt.% | 85 wt.% | 15 wt.% | - | - | - | - |
| Comp. Ex. 6 | 100 Wt% | Co₃O₄ 10 wt.% | YSZ 90 wt.% | 100 wt.% | - | - | - | O | - |

### 1. Evaluation of selectivity to ammonia depending on the type of the first metal oxide

Each of the ammonia gas sensors fabricated in Examples 1 to 10 and Comparative Example 1 was placed under a model gas flow generated by a model gas generation apparatus, and the selectivity to ammonia was assessed. The model gas had a gas temperature of 280°C, and the sensor element portion was controlled to 600°C (heating by a heater). The gas composition was adjusted to O₂=10%, H₂O=5%, CO₂=5%, and N₂=bal.
Specifically, the relationship between NH₃ concentration and output of the ammonia gas sensor was determined, and a concentration conversion equation was made therefrom. Firstly, the potential difference between the reference electrode 4A and the detection electrode 2A was measured under the flow of the model gas (NH₃: 0 ppm) generated by the model gas generation apparatus, to thereby obtain the base electromotive force (base EMF). Then, under the flow of a predetermined amount of a model gas having an NH₃ level varied from 0 to 100 ppm, the potential difference between the reference electrode 4A and the detection electrode 2A was measured, to thereby obtain the electromotive force at measurement (measurement EMF). Then, the base electromotive force was subtracted from the measurement electromotive force (base electromotive force: electromotive force measured in the absence of a gas to be analyzed), whereby the relationship between NH₃ concentration and EMF output of the ammonia gas sensor was determined.
Separately, C₃H₆ (100 ppm) serving as an interference gas was added to a C model gas. Under the flow of the gas mixture, the output (EMF value) of the ammonia gas sensor was measured, and a calculated NH₃ concentration value was obtained by the aforementioned concentration conversion equation. The calculated NH₃ concentration value is an index for the degree of detecting C₃H₆ as ammonia. Thus, the higher the calculated NH₃ concentration value, the higher the degree of detecting C₃H₆ as ammonia. That is, selectivity to ammonia is can be evaluated as low. In contrast, the lower the calculated NH₃ concentration value, the lower the degree of detecting C₃H₆ as ammonia. That is, selectivity to ammonia is can be evaluated as high. In consideration of use in practice, the influence of C₃H₆ is preferably null. However, when the calculated NH₃ concentration value is 5 ppm or lower (accuracy: ± 5 ppm or smaller), selectivity to ammonia can be regarded to be excellent.

FIG. 4 shows the results. In the Examples, each employing an intermediate layer containing the first metal oxide, the calculated NH₃ concentration value was 5 ppm or lower (approximately 2 ppm), indicating reduced effect of the interference gas and enhanced selectivity to ammonia.
In contrast, in Comparative Example 1, employing no intermediate layer interposing between the detection electrode and the solid electrolyte member, the calculated NH₃ concentration value considerably exceeded 5 ppm, indicating that selectivity to ammonia was lowered by the influence of the interference gas.
In Table 1, when the calculated NH₃ concentration value was 5 ppm or lower, the selectivity was rated as "O," whereas when the calculated NH₃ concentration value exceeded 5 ppm, the selectivity was rated as "X."

### 2. Evaluation of ammonia detection sensitivity depending on the type of the first metal oxide

Each of the ammonia gas sensors fabricated in Examples ₁ to 10 and Comparative Examples 2, 3, and 6 was placed under a model gas flow generated by a model gas generation apparatus, and the ammonia detection sensitivity was assessed. The model gas had a gas temperature of 280°C, and the sensor element portion was controlled to 600°C (heating by a heater). The gas composition was adjusted to O₂=10%, H₂O=5%, and N₂=bal.
NH₃ (10 ppm) was added to the model gas, and the output of the ammonia gas sensor (EMF value) was measured under the flow of the model gas.

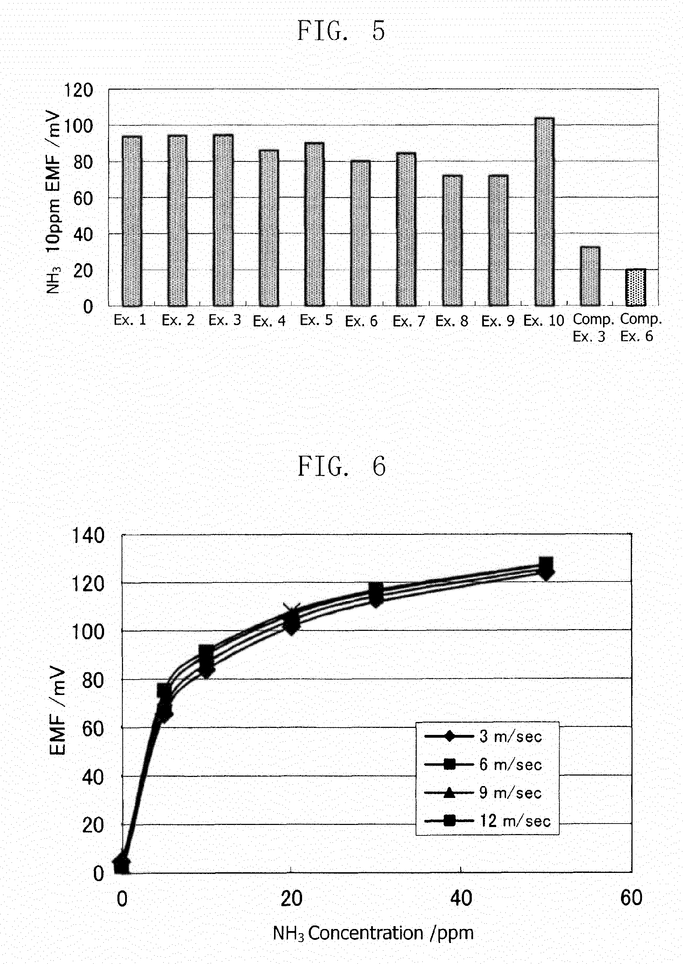

FIG. 5 shows the results. In the Examples, each employing an intermediate layer containing the first metal oxide, the output EMF was 60 mV or higher, indicating that low-level (10 ppm) ammonia can be detected at high sensitivity.
In contrast, in Comparative Example 2, in which the intermediate layer contained no solid electrolyte component, the conductivity of the intermediate layer decreased, to thereby impair electrical conduction between the solid electrolyte and the detection electrode, failing to detect ammonia gas.
In Comparative Example 3, in which no intermediate layer was disposed, and the detection electrode contained the first metal oxide, the output EMF was lower than 60 mV, and detection sensitivity to low-level (10 ppm) ammonia decreased. One conceivable reason for this is that ammonia burnt at the detection electrode, whereby the amount of ammonia reaching the interface between the detection electrode and the intermediate layer was reduced.
In Comparative Example 6, in which the detection electrode contained no YSZ, the detection electrode contained the first metal oxide, the output EMF was lower than 60 mV, and detection sensitivity to low-level (10 ppm) ammonia decreased. One conceivable reason for this is that the detection electrode was excessively densified, due to the absence of YSZ serving as a co-base of the detection electrode, whereby the amount of ammonia reaching the interface between the detection electrode and the intermediate layer was reduced.
In Table 1, when the EMF was 60 mV or higher, the sensitivity was rated as "OO," when the EMF was 20 to 60 mV, the sensitivity was rated as "O," and when the EMF was lower than 20 mV, the sensitivity was rated as "X."

### 3. Evaluation of influence of gas flow speed

Each of the ammonia gas sensors fabricated in Example 4 and Comparative Example 3 was placed under a model gas flow generated by a model gas generation apparatus, and the influence of gas flow speed was assessed. The model gas had a gas temperature of 280°C, and the sensor element portion was controlled to 600°C (heating by a heater). The gas composition was adjusted to O₂=10%, H₂O=5%, and N₂=bal.
Then, NH₃ (5, 10, 20, 30, or 50 ppm) was added to the model gas, and the gas flow speed was varied from 3 to 12 m/sec at each NH₃ concentration. Under the gas flow, the output of the ammonia gas sensor (EMF value) was measured, and the calculated NH₃ concentration value was obtained by the aforementioned concentration conversion equation. The higher the calculated NH₃ concentration value, the higher the ammonia detection degree. That is, the ammonia detection sensitivity can be regarded as high.

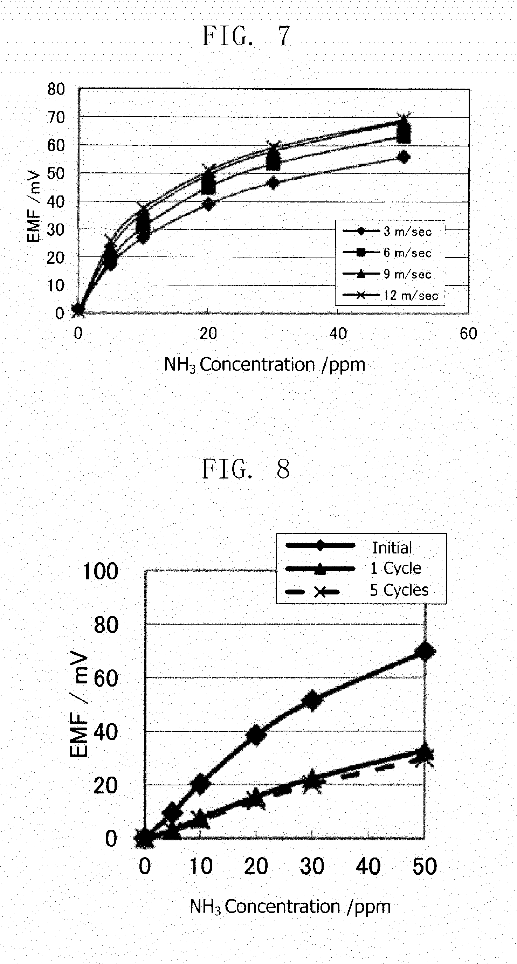

FIGs. 6 and 7 show the results of Example 4 and Comparative Example 3, respectively. At any gas flow speed, the calculated NH₃ concentration value was higher in Example 4 than in Comparative Example 3. The influence of gas flow speed was found to be small in the Example. Although the reason for small influence of gas flow speed in Example 4 has not been clearly elucidated, one conceivable reason is that burning and decomposition of ammonia was prevented in the detection electrode due to the absence of the first metal oxide in the detection electrode, whereby the decrease in amount of ammonia reaching the solid electrolyte member was suppressed.
The ammonia gas sensors of Examples 1 to 3 and 5 to 10 were evaluated in the same manner. In all cases, the influence of gas flow speed was small.
In Table 1, when the variation in EMF with respect to 50 ppm NH₃ within a flow speed range (3 to 12 m/sec) was 5 mV or less, the influence of gas flow speed was rated as "O," whereas when the variation in EMF was 5 mV or more, the influence was rated as "X."

As is clear from Table 1, in Comparative Example 2, in which the intermediate layer contained no solid electrolyte component; in Comparative Example 4, in which the detection electrode had an Au content of less than 70 wt%; and in Comparative Example 5, in which the intermediate layer had a solid electrolyte component content less than 50 wt%, electrical conduction between the solid electrolyte and the detection electrode was impaired, failing to detect ammonia gas.
Therefore, the intermediate layer must have a solid electrolyte component content of 50 wt% or higher, and the detection electrode must have an Au content of 70 wt% or higher.

Separately, the ammonia gas sensors of Examples 11 to 16 were fabricated through the same production method as employed in Example 3 shown in Table 1. However, in Example 3, the paste for forming the intermediate layer was prepared by mixing a solid electrolyte component powder and a first metal oxide powder, while, in Examples 11 to 16, a paste in which the first metal oxide was deposited on the solid electrolyte component powder was used. Specifically, a solid electrolyte component powder (e.g., YSZ powder) as shown in Table 2 was immersed in a predetermined concentration nitrate solution of the first metal oxide (e.g., Co₃O₄) as shown in Table 2, and the solution was vaporized to dryness, to thereby yield a first metal oxide-deposited powder, from which the paste was prepared.

**[Table 2]**

| | YSZ content of solid electrolyte member | Intermediate layer | | | Detection electrode | | | Evaluation |
|---|---|---|---|---|---|---|---|---|
| | | First metal oxide (type/content) | Solid electrolyte component (type/content) | Addition | Au | YSZ | Co₃O₄ | Applicability to high-concentration gas |
| Ex. 3 | 100 wt. % | Co₃O₄ 5 wt.% | YSZ 95 wt.% | Mixing | 85 wt.% | 15 wt.% | - | △ |
| Ex. 11 | 100 wt. % | Co₃O₄ | YSZ 99 wt. % | Deposition | 85 wet. % | 15 wt.% | - | O |
| Ex. 12 | 100 wt. % | Co₃O₄ 3 wt.% | YSZ 97 wt. % | Deposition | 85 wt.% | 15 wet.% | - | O |
| Ex. 13 | 100 wt. % | Co₃O₄ | YSZ 95 wt.% | Deposition | 85 wet. % | 15 wet.% | - | O |
| Ex. 14 | 100 wt. % | Co₃O₄ 10wt.% | YSZ 90 wt.% | Deposition | 85wt.% | 15wt.% | - | O |
| Ex. 15 | 100 wt.% | Co₃O₄ 5wt.% | Ce02 95 wet.% | Deposition | 85 wt.% | 15 wt.% | - | O |
| Ex. 16 | 100 wt.% | MnO 5 wt.% | YSZ 95 wt.% | Deposition | 85 wet.% | 15 wt.% | - | O |

### 4. Evaluation on the applicability to high-concentration gas

Each of the ammonia gas sensors fabricated in Examples 3 and 11 to 16 was placed under a model gas flow generated by a model gas generation apparatus, and the applicability of the sensor to high-concentration gas was assessed through the following procedure.
(1) The sensor element portion was controlled to 600°C (heating by a heater). The gas composition was adjusted to O₂=7%, H₂O=4%, and N₂=bal . Then, NH₃ (0, 5, 10, 20, 30, or 50 ppm) was added to the model gas. Under the gas flow, the output of the ammonia gas sensor (EMF value) was measured (initial value).
(2) Then, while the ammonia gas sensor was continuously attached to the model gas generation apparatus, the gas feed to the model gas generation apparatus was changed to a high-concentration gas having a composition of C₃H₆=4,500 ppm and N₂ bal. The high-concentration gas was continued to flow for 30 minutes.
(3) Thereafter, while the ammonia gas sensor was continuously attached to the model gas generation apparatus, the model gas generation apparatus was changed to a low-concentration gas having a composition of O₂=20% and N₂ bal., and the flow of the low-concentration gas was continued for 5 minutes.
(4) After performance of 1 cycle ((2) to (3)) or 5 cycles, the output of the ammonia gas sensor (EMF value) was measured. The output of the ammonia gas sensor was measured through the same method as employed in (1).

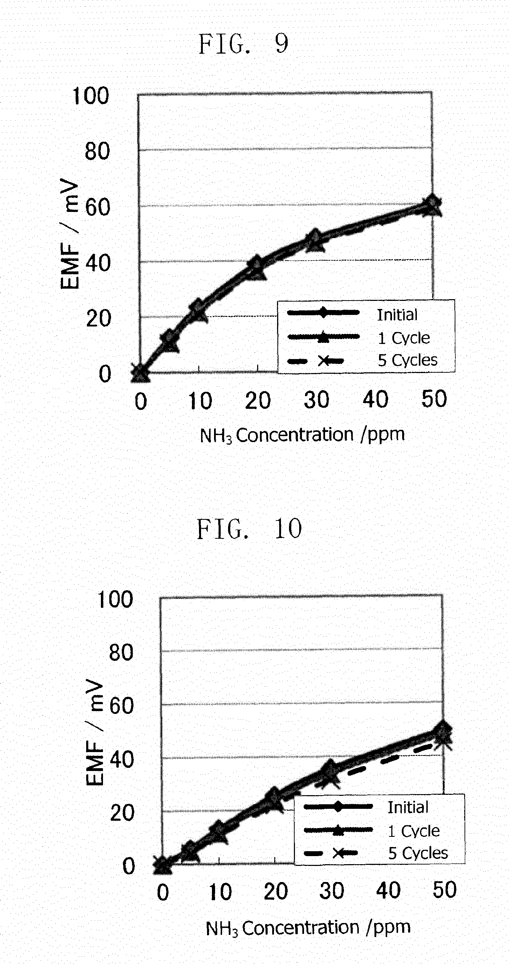

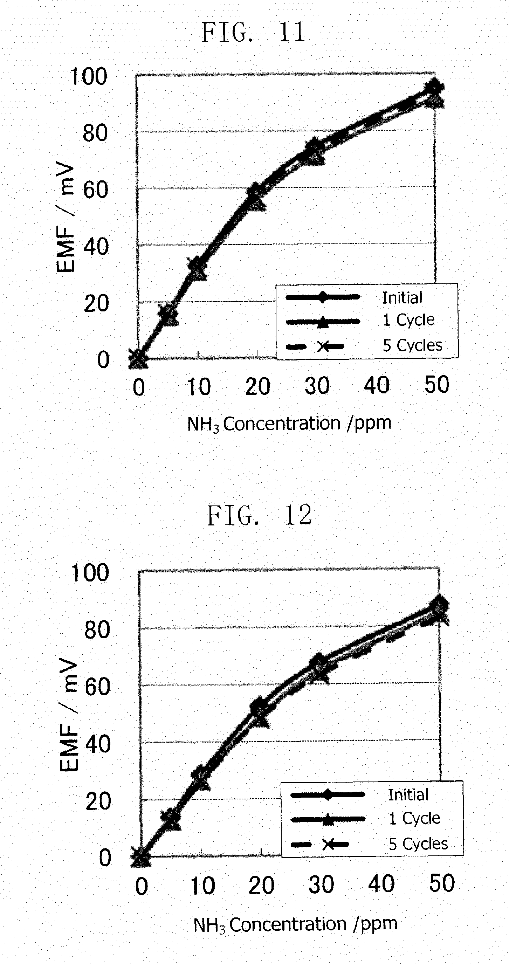

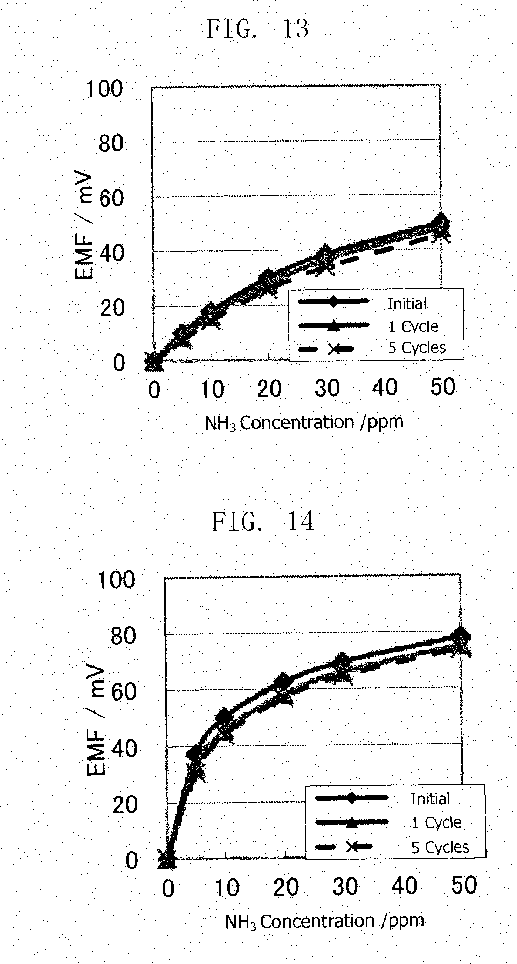

FIGs. 8 to 14 show the results. FIGs. 8 to 14 correspond to Examples 3, and 11 to 16, respectively. Each of the ammonia gas sensors of Examples 11 to 16 exhibited no substantial drop in output with respect to the initial value after performance of 1 cycle and 5 cycles. In contrast, the ammonia gas sensor of Example 3 exhibited a drop in output with respect to the initial value after performance of 1 cycle and 5 cycles.
Therefore, the above tests have revealed that the intermediate layer preferably contains a solid electrolyte component deposited on the first metal oxide.

In Examples 3 and 13, a cross section of the intermediate layer was observed in five areas (3 µm x 3 µm) under an SEM. In Example 3, the state in which Co₃O₄ not bonded to the solid electrolyte component was present was observed in two or more areas. In contrast, in Example 13, the state in which Co₃O₄ not bonded to the solid electrolyte component was present was observed in only one area.

### [Description of Reference Numerals]

- 2A: detection electrode
- 4A: reference electrode
- 5A: intermediate layer
- 22A: solid electrolyte member
- 50A: gas sensor element
- 200A: ammonia gas sensor

## Claims

1. An ammonia gas sensor comprising:
an oxygen ion conductive solid electrolyte member;
a detection electrode and a reference electrode which are disposed on the solid electrolyte member; and
an intermediate layer interposing between the detection electrode and the solid electrolyte member;
wherein the intermediate layer contains the oxygen ion conductive solid electrolyte component in an amount of 50 wt% or more, and a first metal oxide which is an oxide of at least one metal selected from the group consisting of Co, Mn, Cu, Ni, and Ce, and
the detection electrode contains Au in an amount of 70 wt% or higher and no first metal oxide.

2. An ammonia gas sensor according to claim 1, wherein the reference electrode is directly disposed on the solid electrolyte member.

3. An ammonia gas sensor according to claim 1 or 2, wherein the intermediate layer contains the first metal oxide in an amount of 1 to 50 wt%.

4. An ammonia gas sensor according to any one of claims 1 to 3, wherein the intermediate layer comprises the solid electrolyte component deposited on the first metal oxide.

5. An ammonia gas sensor according to any one of claims 1 to 4, wherein the detection electrode is a porous electrode containing a second metal oxide which is an oxide of at least one metal selected from the group consisting of Zr, Y, Al, and Si.

6. An ammonia gas sensor according to any one of claims 1 to 5, wherein the first metal oxide is Co₃O₄.

7. An ammonia gas sensor according to any one of claims 1 to 6, wherein the intermediate layer is porous.
